(19)

**Europäisches Patentamt**
**European Patent Office**
**Office européen des brevets**

(11)  **EP 3 849 668 B1**

(12)  # FASCICULE DE BREVET EUROPEEN

(45) Date de publication et mention
de la délivrance du brevet:
**19.07.2023   Bulletin 2023/29**

(51) Classification Internationale des Brevets (IPC):
**A61Q 7/00** (2006.01)    **A61K 36/22** (2006.01)
**A61K 36/67** (2006.01)    **A61K 8/9789** (2017.01)

(21) Numéro de dépôt: **19790640.7**

(22) Date de dépôt: **13.09.2019**

(52) Classification Coopérative des Brevets (CPC):
(C-Sets disponibles)
**A61K 8/9789; A61K 36/22; A61K 36/67; A61Q 7/00**

(Cont.)

(86) Numéro de dépôt international:
**PCT/FR2019/052128**

(87) Numéro de publication internationale:
**WO 2020/053531 (19.03.2020 Gazette 2020/12)**

(54) **UTILISATION COSMÉTIQUE D'EXTRAITS VÉGÉTAUX**

VERWENDUNG VON PFLANZENEXTRAKTEN IN DER KOSMETIK

USE OF PLANT EXTRACTS IN COSMETICS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **13.09.2018   FR 1858218**

(43) Date de publication de la demande:
**21.07.2021   Bulletin 2021/29**

(73) Titulaire: **Robertet S.A.**
**06130 Grasse (FR)**

(72) Inventeur: **PEGARD, Anthony**
**06130 GRASSE (FR)**

(74) Mandataire: **Germain Maureau**
**12, rue Boileau**
**69006 Lyon (FR)**

(56) Documents cités:
**JP-A- H10 265 340      US-A1- 2009 324 760**

• **DATABASE WPI Week 200331 1 août 2013
(2013-08-01) Thomson Scientific, London, GB;
AN 2003-317461 XP002791539, & JP 2002 179582
A (UNIV SHANGHAI CHINESE MEDICINE) 26 juin
2002 (2002-06-26)**

• **DATABASE GNPD [Online] MINTEL; 11 juin 2018
(2018-06-11), anonymous: "Riah Hair Oil",
XP055590853, extrait de www.gnpd.com
Database accession no. 5718587**
• **Satish Patel ET AL: "Hair Growth: Focus on
Herbal Therapeutic Agent", Current Drug
Discovery Technologies, 1 janvier 2015
(2015-01-01), pages 21-42, XP055591061, Extrait
de l'Internet:
URL:https://www.researchgate.net/profile/N
agendra_Chauhan/publication/278045537_Hair
_Growth_Focus_on_Herbal_Therapeutic_Agent
/ links/55af72fd08ae6aa568b3a960/Hair-Growth
-Focus-on-Herbal-Therapeutic-Agent.pdf [extrait
le 2019-05-22]**
• **DATABASE GNPD [Online] MINTEL; 23 août 2018
(2018-08-23), anonymous: "Nature's Hair Miracle
Strengthening & Energizing Hair Oil",
XP055590899, extrait de www.gnpd.com
Database accession no. 5926059**
• **Noriko Hirata ET AL: "Testosterone
5a-Reductase Inhibitory Active Constituents of
Piper nigrum Leaf", Biol. Pharm. Bull. 30(12), 1
janvier 2007 (2007-01-01), pages 2402-2405,
XP055591071, Extrait de l'Internet:
URL:https://www.jstage.jst.go.jp/article/b
pb/30/12/30_12_2402/_article/-char/ja/ [extrait le
2019-05-22]**

**(Cont. page suivante)**

- "Triphalaghrtam", TKDL,, 1 janvier 1999 (1999-01-01), XP003029818,
- DATABASE WPI Week 199812 Thomson Scientific, London, GB; AN 1998-126063 XP002791540, & JP H10 7535 A (SHISEIDO CO LTD) 13 janvier 1998 (1998-01-13)
- DATABASE GNPD [Online] MINTEL; 14 août 2018 (2018-08-14), anonymous: "Beard Wash", XP055590988, extrait de www.gnpd.com Database accession no. 5898691
- DATABASE WPI Week 200918 1 avril 2008 (2008-04-01) Thomson Scientific, London, GB; AN 2009-B48145 XP002791541, & KR 100 848 800 B1 (BIO SPECTRUM INC) 31 juillet 2008 (2008-07-31)
- DATABASE GNPD [Online] MINTEL; 2 août 2018 (2018-08-02), anonymous: "Shampoo", XP055590996, extrait de www.gnpd.com Database accession no. 5859887
- DASGUPTA A ET AL: "MEDICINAL SPECIES OF PIPER, PHARMACOGNOSTIC DELIMITATION", QUARTERLY JOURNAL OF CRUDE DRUG RESE, SWETS AND ZEITLINGER, LISSE, NL, vol. 18, no. 1, 1 janvier 1980 (1980-01-01), pages 17-25, XP009006829, ISSN: 0033-5525
- M A Sumathykutty ET AL: "Essential oil constituents of some Piper species", Flavour and Fragrance Journal, 14, 1 janvier 1999 (1999-01-01), pages 279-282, XP055591044, Extrait de l'Internet: URL:https://onlinelibrary.wiley.com/doi/abs/10.1002/(SICI)1099-1026(199909/10)14:5%3C279::AID-FFJ821%3E3.0.CO;2-0 [extrait le 2019-05-22]
- HOUCINE BENDAOUD ET AL: "Chemical Composition and Anticancer and Antioxidant Activities of?Schinus Molle?L. and?Schinus?Terebinthifolius?Raddi Berries Essential Oils", JOURNAL OF FOOD SCIENCE, vol. 75, no. 6, 1 août 2010 (2010-08-01), pages C466-C472, XP055591081, US ISSN: 0022-1147, DOI: 10.1111/j.1750-3841.2010.01711.x

(52) Classification Coopérative des Brevets (CPC): (Cont.)

C-Sets
A61K 36/22, A61K 2300/00;
A61K 36/67, A61K 2300/00

**Description**

La présente invention a pour objet l'utilisation cosmétique d'extraits végétaux pour favoriser la pousse des poils.

Chez l'homme, on cherche à favoriser la pousse des poils sur différentes parties du corps pour des raisons essentiellement esthétiques.

Dans le cadre de la présente invention le terme « poils » désignera indifféremment les cheveux ou les poils, comme les poils de barbe.

Poils et cheveux sont faits par l'assemblage de cellules produites dans le follicule par kératinisation, le follicule pileux étant la cavité dans laquelle ils prennent naissance.

La principale différence entre les poils et les cheveux réside dans leur croissance. Alors que le cheveu pousse indéfiniment, en continu, le poil stoppe sa croissance une fois sa grandeur maximale atteinte, puis est remplacé par un semblable.

Les végétaux sont une source notoire d'extraits et d'ingrédients actifs permettant de stimuler la pousse des cheveux.

Ainsi, l'objet du document KR 100848800 B1 est une composition cosmétique à base d'huile de poivre du Brésil (aussi appelé baies roses), extraite d'une plante de l'espèce *Schinus terebinthifolius* pour, entre autres propriétés, traiter la chute des cheveux et favoriser leur pousse.

On connaît aussi un shampoing « Cosmeceuta Hortelã Pimenta » (Mintel GNPD, accession no 5859887) notamment pour lutter contre la chute des cheveux, comprenant des extraits de plantes dont un extrait de fruits de *Schinus terebinthifolius.*

Selon H. Bendaoud et al. Journal of Food Science (2010) vol. 75, no 6 pages C466-C472, les auteurs ont analysé les huiles extraites des fruits des espèces *Schinus molle* et *Schinus terebinthifolius* et investigué leurs activités antioxydantes et anticancéreuses.

JPH107535A divulgue l'utilisation cosmétique d'un extrait éthanolique de Cabe jawa (ou *Piper retrofractum*) pour favoriser la pousse des cheveux.

Le document JP H10265340 A décrit des dérivés du 1,3-benzodioxole de préférence isolés de plantes telles que celles de la famille des *Piperaceae,* et notamment des espèces *Piper retrofractum vahl* et *Piper nigrum L.,* pour stimuler la pousse du cheveu et prévenir l'alopécie.

Selon le document N. Hirata et al., Biol. Pharm. Bull. 30(12) (2007) pp 2402-2405, on connaît l'utilisation cosmétique d'extraits de *Piper nigrum* pour traiter à la fois l'alopécie, par inhibition de la testotérone-5-alpha-réductase, et la dépigmentation des cheveux par stimulation de la mélanogénèse.

US2009/324760A1 décrit l'utilisation de l'actéoside à titre d'ingrédient actif dans une composition cosmétique pour favoriser la pousse du cheveu et traiter l'alopécie. L'actéoside est présent dans de nombreuses plantes dont celles de la famille des Piperaceae à partir desquelles il peut être obtenu par extraction puis purification.

JP 2002-1979582A décrit l'utilisation d'un extrait de *Fructus litseae* qui semble désigner les fruits de *Piper cubeba,* pour inhiber la testotérone-5-alpha-réductase. Cet extrait est obtenu par extraction aqueuse ou dans un solvant tel que le méthanol et peut être utilisé dans une composition cosmétique pour faire pousser les cheveux.

On connaît une huile capillaire, RIAH Hair Oil (Mintel GNPD, no accession 5718587), préparée à base de 42 extraits de plantes dont *Piper cubeba,* notamment dans une indication pour la pousse des cheveux.

S. Patel et al., Current Drug Discovery Technologies (2015) pages 21-42 présente différentes plantes dont les extraits possèdent un effet stimulant sur la pousse des cheveux, dont un extrait hydroéthanolique de feuilles de *Piper nigrum.*

Selon la base de données Mintel GNPD (no accession 5926059), on connaît une huile capillaire, Natural 's Hair Miracle Strenghtening & Energizing Hair Oil, comprenant entre autres extraits végétaux, un extrait de fruits de *Piper nigrum,* pour renforcer le cheveu.

Dans la base de données TKDL, on trouve une composition (Triphalaghrtam) appartenant à un savoir traditionnel de la médecine ayurvédique, pour traiter l'alopécie et la canitie, comprenant entre autres ingrédients, une fraction de *Piper nigrum* Linn et une fraction de *Piper longum Linn.*

Le produit cosmétique « MAN CAVE Blackspice Beard Wash » destiné à nettoyer, fortifier et hydrater la barbe, et comprenant entres autres ingrédients, de l'huile de fruits de *Piper nigrum* et de l'huile de Schinus molle, est divulgué dans la base de données Mintel GNPD (no accession 5898691).

L'article A. Dasgupta et al., Quarterly Journal ofCrude Drug Ree, Swets and Zeitlinger, Lisse, NL (1980) vol. 18, no 1, pages 17-25 traite des utilisations médicinales d'extraits de plantes du genre Piper. Ainsi, l'huile extraite des fruits de l'espèce *Piper cubeba* est décrite pour soigner des maladies génito-urinaires, l'acide cubébique présent dans l'huile en serait l'ingrédient actif.

Selon MA Sumathykutty et al., Flavour and Fragrances Journal (1999) 14, pages 279-282, les auteurs ont analysé les huiles extraites de différentes espèces du genre Piper et en rapportent la composition. L'huile extraite des fruits de *Piper cubeba* contient essentiellement des sesquiterpènes et des monoterpènes (dont le β-pinène).

Le follicule pileux constitue l'unité biologique fondamentale, directement impliquée dans le développement et le maintien du système pilaire. Il rassemble différentes entités structurales telles que la papille dermique, la glande sébacée, les gaines épithéliales, l'innervation et la vascularisation, impliquées séparément ou conjointement dans le cycle de développement des cheveux.

Par rapport à l'art antérieur ci-dessus, l'invention apporte des extraits végétaux qui agissent directement sur le follicule pileux et plus précisément dans la papille dermique, permettant de favoriser la pousse des poils et ce, quel que soit l'état du sujet traité, en particulier qu'il soit atteint d'alopécie ou de toute autre forme de chute ou de faiblesse des poils, ou non.

Ainsi, la présente invention a notamment pour objet l'utilisation cosmétique d'au moins deux extraits végétaux, pour favoriser la pousse des poils. Ces extraits sont obtenus à partir d'au moins deux végétaux choisis parmi *Piper cubeba, Piper nigrum* et une plante du genre *Schinus,* et en particulier l'espèce *Schinus terebenthifolius.*

En effet, de façon surprenante, il a été trouvé qu'une combinaison d'au moins deux extraits végétaux, avantageusement de deux ou trois extraits végétaux, choisis parmi les extraits obtenus à partir d'au moins deux plantes choisies parmi l'espèce *Piper cubeba,* l'espèce *Piper nigrum* et l'espèce *Schinus terebenthifolius* a une action favorisant la pousse des poils. Comme cela sera plus loin démontré, un effet synergique des extraits entre eux sur la pousse des poils est observé.

Avant d'exposer plus en détails l'invention, certains termes employés dans le présent texte sont définis.

Dans le cadre de la présente invention on entend par « favoriser la pousse » une augmentation de la densité et/ou de l'épaisseur du poil.

L'espèce *Piper cubeba* est aussi appelée cubèbe ; l'espèce *Piper nigrum* est aussi appelée poivre noir ou poivrier noir; et l'espèce *Schinus terebenthifolius* est encore connue sous les noms de poivre rose et baies roses.

On utilisera indifféremment les termes composition, combinaison, association pour désigner un mélange d'au moins deux extraits utilisés selon l'invention. On désignera par formulation, tout mélange d'au moins deux extraits tels que définis dans la présente invention avec toute(s) autre(s) matière(s), par exemple un ou plusieurs autres ingrédients actifs différents des extraits de l'invention contribuant à l'effet recherché ou apportant une autre activité ; un ou plusieurs additifs comme un parfum, un conservateur, un antioxydant ; un ou plusieurs solvants ; un ou plusieurs excipients ; un ou plusieurs adjuvants ; et ce, quelle que soit la forme de la ou desdites matières, notamment solide, liquide, et quelle que soit son origine, naturelle ou synthétique. Dans le cadre de l'invention, cette ou ces matières sont dermatologiquement ou cosmétiquement acceptables.

Dans la description les termes « l'extrait est obtenu à partir d'une plante », « l'extrait est obtenu à partir d'un végétal », et « extrait de » ont la même signification.

L'efficacité des extraits végétaux de l'invention sur la densité et/ou l'épaisseur du poil est mesurée comparativement à un contrôle par des méthodes bien connues de l'homme du métier comme par exemple, à l'aide d'un vidéomicroscope.

Il est également possible d'évaluer l'efficacité des combinaisons d'extraits en étudiant la densité pilaire (nombre total de poils par cm$^2$), la vitesse de pousse des poils (en mm par jour) ou la variation de l'épaisseur des poils par scorage ou encore par l'étude de l'effet des compositions sur des cellules de la papille dermique de follicules humains (HFDPC, Human Follicule Dermal Papilla Cells) et plus particulièrement par l'effet des compositions sur la quantité de facteur de croissance endothélial vasculaire (VEGF) synthétisée.

Dans le cadre de la présente invention les extraits végétaux de la présente invention sont des extraits utilisés en parfumerie, comme les huiles essentielles, les concrètes, les résinoïdes, les absolues, les extraits au CO$_2$ supercritique ou encore les extraits obtenus avec des solvants polaires comme les extraits alcooliques, hydro-alcooliques, glycoliques ou glycérinés. Ils sont préparés selon des techniques bien connues de l'homme du métier.

Dans un aspect particulier, l'invention a pour objet l'utilisation cosmétique d'un extrait de *Piper nigrum* et un extrait *de Schinus terebenthifolius,* d'un extrait de *Piper cubeba* et un extrait *de Schinus terebenthifolius et* d'un extrait de *Piper nigrum* et un extrait de *Piper cubeba.* Dans un autre aspect, l'invention concerne l'utilisation cosmétique d'un extrait de *Piper nigrum,* un extrait de *Piper cubeba* et un extrait *Schinus terebenthifolius* pour favoriser la pousse des poils.

Dans le cadre de la présente invention, le(s) extrait(s) sont présents en quantité efficace pour favoriser la pousse des poils.

L'homme du métier saura définir la stoechiométrie de chacun des extraits végétaux présents dans la composition de l'invention.

Dans une mise en oeuvre particulière de l'invention, les extraits pourront être présents en poids identique (1/1 pour deux extraits ou 1/1/1 pour trois extraits) ; dans une autre mise en oeuvre les extraits pourront être présents en quantités différentes.

Dans un aspect particulier, les extraits végétaux peuvent être associés à au moins un autre composé, et notamment au moins un composé odorant, au moins un antioxydant et/ou au moins un solvant.

Les composés odorants sont bien connus de l'homme du métier dans le domaine de la cosmétologie. Ces composés sont présents dans une variété de classes chimiques, mais sont en général des composés volatiles et insolubles dans l'eau. Ces composés odorants se trouvent dans des extraits végétaux tels que, des huiles essentielles, des absolues, des résinoïdes, des extraits $CO_2$ ou sont des produits de synthèse. Ces composés sont présents en quantité suffisante pour fournir une odeur agréable qui peut être perçue par un consommateur.

Les solvants susceptibles d'être utilisés sont également bien connus de l'homme du métier, on peut citer à titre d'exemples le myristate d'isopropyle, le benzoyl benzoate, le triéthyl citrate ou le diéthyl phtalate, des solvants polaires l'eau, l'éthanol, le glycérol, l'eau glycérinée, les éthers de glycol tels que le propylène-glycol, butylène-glycol, le dipropylène glycol (DPG), employés seuls ou en mélanges.

Le(s) antioxydants(s) susceptible(s) d'être utilisé(s) sont également bien connu(s) de l'homme du métier, on peut citer à titre d'exemples le tocophérol, l'hydroxytoluène butylé (BHT), le Trolox.

Dans une mise en oeuvre particulière de l'invention, la combinaison d'extraits représente de préférence de 10 ppm à 20% en poids par rapport au poids total de la formulation cosmétique. Selon une autre mise en oeuvre particulière, la combinaison d'extraits représente de 1% à 35% en poids par rapport au poids total de la formulation cosmétique.

La combinaison d'extraits peut être utilisée dans une formulation cosmétique comprenant ladite combinaison selon l'invention. Une telle formulation cosmétique comprend toute autre matière dermatologiquement ou cosmétiquement acceptable.

Cette ou ces matières dermatologiquement ou cosmétiquement acceptables sont bien connues de l'homme du métier.

La formulation cosmétique peut être, par exemple, un produit cosmétique ou dermatologique, sous la forme d'une pommade, d'une lotion, d'une mousse, d'une crème, d'un gel, d'une solution, d'une émulsion huile dans eau ou eau dans huile, d'un onguent, d'une huile corporelle, etc.

La formulation cosmétique peut aussi prendre la forme d'une lotion ou d'une solution dans laquelle la combinaison selon l'invention est sous forme encapsulée. La combinaison selon l'invention peut être incorporée dans des vecteurs de type liposomes, glycosphères, cyclodextrines, dans des chylomicrons, des macro-, micro-, nano-particules ainsi que les macro-, micro- et nanocapsules et aussi être absorbés sur des polymères organiques poudreux, les talcs, bentonites et autres supports minéraux.

La formulation cosmétique peut prendre la forme de gel comprenant des excipients appropriés tels que les esters de cellulose ou d'autres agents gélifiants, tels que le carbopol, le sepinov (polyacrylate), la gomme guar, etc.

La formulation cosmétique peut aussi contenir des additifs ou des adjuvants usuels en cosmétologie comme par exemple des agents antimicrobiens mais aussi des lipides d'extraction ou de synthèse, des polymères gélifiants et viscosifiants, des tensio-actifs et des émulsifiants, des principes actifs hydro- ou liposolubles, des extraits de plantes, des extraits tissulaires, des extraits marins, des actifs de synthèse.

La formulation cosmétique peut aussi comprendre d'autres principes actifs complémentaires choisis pour leur action, par exemple pour l'effet hydratant, l'effet anti-âge, l'activité antimicrobienne, l'activité antioxydante, l'effet cicatrisant, l'effet tenseur, l'effet anti-ride, l'activité chélatante, l'activité complexante et séquestrante, l'effet apaisant, l'effet anti-rougeurs, l'activité émolliente, l'activité participant au renouvellement cellulaire, mais également la protection solaire, l'activité anti-irritante, la nutrition cellulaire, la respiration cellulaire, les traitements anti-séborrhéiques, la tonicité cutanée.

L'homme du métier saura définir les quantités efficaces à utiliser en fonction du type de produit cosmétique comprenant la composition synergique selon l'invention; par exemple, les extraits végétaux utilisés classiquement dans les produits rincés (par exemple les shampoings, lotions capillaires) seront présents à hauteur d'environ 1% en poids par rapport au poids total de la formulation cosmétique.

Lorsque la formulation cosmétique contient des principes actifs complémentaires, ceux-ci sont généralement présents à une concentration suffisamment élevée pour qu'ils puissent exercer leur activité.

Les formulations cosmétiques sont de préférence utilisées quotidiennement et appliquées une ou plusieurs fois par jour.

Cette utilisation peut être renouvelée jusqu'à l'obtention d'un résultat acceptable par l'utilisateur.

Les formulations sont très bien tolérées, elles ne présentent aucune toxicité et leur application sur la peau, pour des périodes de temps prolongées, n'impliquent aucun effet systémique.

La présente invention peut aussi être utilisée dans une méthode pour favoriser la pousse des poils comprenant la sélection d'une zone de peau sur laquelle la pousse et/ou la repousse des poils est recherchée, et l'application sur ladite zone d'une formulation cosmétique contenant au moins deux extraits précités, en quantité suffisante pour réduire la favoriser la pousse des poils.

La zone de peau sélectionnée peut être située sur le cuir chevelu, le visage, les jambes, le pubis, le torse, les bras ou les aisselles.

La présente invention est illustrée de manière non limitative par les exemples suivants.

### *Exemple 1 : Test de viabilité cellulaire*

Un test de viabilité cellulaire au sel de tétrazolium MTT (bromure de 3-(4,5-dimethylthiazol-2-yl)-2,5-diphenyl tetrazolium) est effectué. Le test MTT est une méthode de numération des cellules vivantes, bien connue de l'homme du métier.

Pour cela les cellules sont incubées pendant 3h avec du milieu contenant 1mg/ml de MTT, avant de solubiliser les cristaux de formazan formés par du DMSO et lire l'absorbance à 570nm. Le taux de survie cellulaire est exprimé en pourcentage par rapport aux contrôles négatifs. Il est mesuré en présence d'une quantité d'extraits selon l'invention correspondant à leur CE50 (cf. exemple 2).

Les résultats sont présentés dans le tableau 1 ci-dessous.

Tableau 1 : test MTT d'extraits végétaux utilisés dans des combinaisons selon l'invention

| Ingrédient (proportion) | % de survie cellulaire à la CE50 |
|---|---|
| Huile Essentielle de Poivre noir *(Piper nigrum)* | 100 |
| Extrait $CO_2$ de Poivre de Cubebe *(Piper cubeba)* | 89 |
| Extrait $CO_2$ de Baies roses (*Schinus terebenthifolius*) | 97 |
| Mélange Poivre noir/ Cubebe (1/1) *(Piper nigrum / Piper cubeba)* | 93 |
| Mélange Poivre noir / Baies roses (1/1) *(Piper nigrum / Schinus terebenthifolius)* | 100 |
| Mélange Cubebe / Baies roses (1/1) *(Piper cubeba / Schinus terebenthifolius)* | 90 |
| Mélange Cubebe / Baies roses / Poivre noir (1/1/1) *(Piper cubeba / Schinus terebenthifolius / Piper nigrum)* | 92 |

Il ressort que les extraits selon l'invention ne manifestent pas de toxicité.

### Exemple 2 : Etude de la quantité de VEGF produite par les cellules en présence de différents extraits végétaux

Plusieurs facteurs sont responsables de la croissance des cheveux, un des plus importants étant le taux de VEGF présent dans les papilles dermiques du cuir chevelu.

Cette cytokine est surtout connue pour son activité angiogénique. Elle augmente la perméabilité vasculaire et par là, l'irrigation du tissu, avec comme conséquence, la croissance du poil (Yano et al., « Control of hair growth and follicle size by VEGF-mediated angiogenesis », J. Clin. Invest., 2001, 107, p. 409-417).

Le VEGF permet, par l'augmentation de l'irrigation sanguine du tissu capillaire, une meilleure nutrition de la base du follicule. Les observations liant le VEGF à la croissance des poils sont nombreuses.

Le test repose sur l'étude de l'effet du produit testé sur des cellules de la papille dermique de follicules humains (HFDPC) et l'étude permet d'évaluer la quantité de facteur de croissance endothélial vasculaire (VEGF) en présence de différentes compositions selon l'invention.

Des tests sur modèle *in vivo* sur cellules HFDPC ont été nécessaires. Ce sont des lignées primaires de cellules de papilles dermiques folliculaires humaines se trouvant à la base des follicules pileux. Elles sont cultivées dans du milieu complet composé de milieu de croissance cellulaire de follicule de papille dermique (FDPCGM), sérum de veau foetal 4 %, extrait d'hypophyse bovine 0.4 %, facteur de croissance basique des fibroblastes humain recombinant 1ng/ml et insuline humaine recombinante 5 μg/mL.

Pour les expériences, les HFDPC sont ensemencées dans des plaques 24 puits, à une densité de 16.000 cellules/cm$^2$ et incubées 24h à 37°C, 5 % $CO_2$. Les cellules sont ensuite mises en présence de milieu contenant les différentes concentrations en matière première (entre 0 et 100 ppm) pour une incubation de 24h. Après ce temps, les surnageants cellulaires sont récupérés dans des microtubes pour des dosages protéiques; tandis que des tests de cytotoxicité sont réalisés sur les cellules (cf. exemple 1).

Le dosage de VEGF-α est réalisé grâce à un test ELISA (PromoKine).

Les résultats sont présentés dans le tableau 2 ci-dessous.

Tableau 2 : Tests sur HFDPC avec évaluation de l'activité de synthèse de VEGF

| Ingrédient (proportion) | CE50 effective (ppm) | CE50 théorique (ppm) |
|---|---|---|
| Huile essentielle de Poivre noir *(Piper nigrum)* | 38 | - |
| Extrait $CO_2$ de Poivre de Cubebe *(Piper cubeba)* | 25 | - |

(suite)

| Ingrédient (proportion) | CE50 effective (ppm) | CE50 théorique (ppm) |
|---|---|---|
| Extrait $CO_2$ de Baies Roses *(Schinus terebenthifolius)* | 76 | - |
| Mélange Poivre noir/ Cubebe (1/1) *(Piper nigrum / Piper cubeba))* | 16 | 30 |
| Mélange Poivre noir/ Baies Roses (1/1) *(Piper nigrum /Schinus terebenthifolius)* | 30 | 51 |
| Mélange Poivre Cubebe/ Baies Roses (1/1) *(Piper cubeba / Schinus terebenthifolius)* | 24 | 38 |
| Mélange Poivre noir/ Cubebe/ Baies Roses (1/1/1) *(Piper nigrum / Piper cubeba / Schinus terebenthifolius)* | 25 | 38 |

La CE50 effective est la concentration d'extrait (en ppm) efficace pour l'obtention d'une activité de synthèse de VEGF supérieure de 50% au témoin (i.e. culture sans extrait).

La CE50 théorique est la valeur de CE50 qui aurait été obtenue en l'absence de synergie entre les extraits (moyenne des CE50 des extraits dans le mélange). Elle est obtenue à l'aide d'un logiciel qui la calcule par extrapolation de l'équation suivante de détermination de la $CI_{50}$ d'un mélange :

$$100 / CI_{50}\text{mélange} = \sum Ci / CI_{50}i*.$$

dans laquelle

i = composant individuel du mélange, variant de 1 à n
Ci = concentration du composant i
n = nombre de composants dans le mélange
$CI_{50}i$ = estimation de la $CI_{50}$ du composant i.
* https://clp-info.ineris.fr/reglementation-clp

Les résultats présentés attestent de l'effet, notamment de l'effet synergique, des extraits sur la quantité de VEGF sécrété par les cellules traitées et par conséquent sur la pousse des poils.

**Revendications**

1.   Utilisation cosmétique d'au moins deux extraits obtenus à partir de deux végétaux choisis parmi *Piper cubeba, Piper nigrum* et *Schinus terebenthifolius,* pour favoriser la pousse des poils.

2.   Utilisation cosmétique selon la revendication 1 d'au moins un extrait obtenu à partir de *Piper nigrum* et un extrait obtenu à partir de *Piper cubeba.*

3.   Utilisation cosmétique selon la revendication 1 ou 2 d'au moins un extrait obtenu à partir de *Piper nigrum* et un extrait obtenu à partir de *Schinus terebenthifolius.*

4.   Utilisation cosmétique selon l'une quelconque des revendications 1 à 3 d'au moins un extrait obtenu à partir *de Piper cubeba* et un extrait obtenu à partir de *Schinus terebenthifolius.*

5.   Utilisation cosmétique selon l'une quelconque des revendications 1 à 4 d'au moins un extrait obtenu à partir de *Piper nigrum,* un extrait obtenu à partir de *Piper cubeba* et un extrait obtenu à partir de *Schinus terebenthifolius.*

6.   Utilisation selon l'une des revendications précédentes, **caractérisée en ce que** les extraits sont des extraits utilisés en parfumerie, comme les huiles essentielles, les concrètes, les résinoïdes, les absolues, les extraits au $CO_2$ super critiques ou les extraits par solvants aqueux.

7.  Utilisation cosmétique telle que défini dans l'une des revendications 1 à 6 dans laquelle les extraits sont présents en poids identiques ou différents pour favoriser la pousse des poils.

**Patentansprüche**

1.  Kosmetische Verwendung von mindestens zwei Extrakten, die aus zwei Pflanzen gewonnen werden, ausgewählt aus *Piper cubeba, Piper nigrum* und *Schinus terebenthifolius,* zur Förderung des Haarwuchses.

2.  Kosmetische Verwendung nach Anspruch 1 von mindestens einem aus *Piper nigrum* gewonnenen Extrakt und einem aus *Piper cubeba* gewonnenen Extrakt.

3.  Kosmetische Verwendung nach Anspruch 1 oder 2 von mindestens einem aus *Piper nigrum* gewonnenen Extrakt und einem aus *Schinus terebenthifolius* gewonnenen Extrakt.

4.  Kosmetische Verwendung nach einem der Ansprüche 1 bis 3 von mindestens einem aus *Piper cubeba* gewonnenen Extrakt und einem aus *Schinus terebenthifolius* gewonnenen Extrakt.

5.  Kosmetische Verwendung nach einem der Ansprüche 1 bis 4 von mindestens einem aus *Piper nigrum* gewonnenen Extrakt, einem aus *Piper cubeba* gewonnenen Extrakt und einem aus *Schinus terebenthifolius* gewonnenen Extrakt.

6.  Verwendung nach einem der vorstehenden Ansprüche, **dadurch gekennzeichnet, dass** es sich bei den Extrakten um Extrakte handelt, die in der Parfümerie verwendet werden, wie etwa ätherische Öle, Concrètes, Resinoide, Absolues, Extrakte mit überkritischem $CO_2$ oder Extrakte mit wässrigen Lösungsmitteln.

7.  Kosmetische Verwendung wie in einem der Ansprüche 1 bis 6 definiert, wobei die Extrakte in gleichen oder unterschiedlichen Gewichten vorliegen, zur Förderung des Haarwuchses.

**Claims**

1.  A cosmetic use of at least two extracts obtained from two plants selected from *Piper cubeba, Piper nigrum* and *Schinus terebenthifolius,* to promote the hair growth.

2.  The cosmetic use according to claim 1 of at least one extract obtained from *Piper nigrum* and one extract obtained from *Piper cubeba.*

3.  The cosmetic use according to claim 1 or 2 of at least one extract obtained from *Piper nigrum* and one extract obtained from *Schinus terebenthifolius.*

4.  The cosmetic use according to any one of claims 1 to 3 of at least one extract obtained from *Piper cubeba* and one extract obtained from *Schinus terebenthifolius.*

5.  The cosmetic use according to any one of claims 1 to 4 of at least one extract obtained from *Piper nigrum,* one extract obtained from *Piper cubeba* and one extract obtained from *Schinus terebenthifolius.*

6.  The use according to any of the preceding claims, **characterized in that** the extracts are extracts which are used in perfumery, such as essential oils, concretes, resinoids, absolutes, supercritical $CO_2$ extracts or aqueous solvent extracts.

7.  The cosmetic use as defined in any of claims 1 to 6 wherein the extracts are present in identical or different weights to promote the hair growth.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- KR 100848800 B1
- JP H10265340 A
- US 2009324760 A1
- JP 20021979582 A

**Littérature non-brevet citée dans la description**

- **SELON H. BENDAOUD et al.** *Journal of Food Science,* 2010, vol. 75 (6), C466-C472
- **N. HIRATA et al.** *Biol. Pharm. Bull.,* 2007, vol. 30 (12), 2402-2405
- **S. PATEL et al.** *Current Drug Discovery Technologies,* 2015, 21-42
- **A. DASGUPTA et al.** Quarterly Journal ofCrude Drug Ree. Swets and Zeitlinger, 1980, vol. 18, 17-25
- **MA SUMATHYKUTTY et al.** Flavour and Fragrances Journal. 1999, vol. 14, 279-282
- **YANO et al.** Control of hair growth and follicle size by VEGF-mediated angiogenesis. *J. Clin. Invest.,* 2001, vol. 107, 409-417